# EUROPÄISCHE PATENTANMELDUNG

(11) **EP 4 574 011 A1**
(43) Veröffentlichungstag der Anmeldung: **25.06.2025**
(21) Anmeldenummer: 24219575.8
(22) Anmeldetag: 12.12.2024
(51) Int. Cl.: A61B 1/00, A61B 1/005, A61B 1/008, A61M 25/01, A61B 17/00

(54) **ARTIKULATIONSGLIED FÜR EINE ODER IN EINER AUSLENKMECHANIK**

(30) Priorität: 20.12.2023 DE 102023135953
(71) Anmelder: Karl Storz SE & Co. KG, 78532 Tuttlingen (DE)
(72) Erfinder: LANDER, Leonik, 78532 Tuttlingen (DE); FUCHS, Alexander, 78532 Tuttlingen (DE); KARL, Wolfgang, 78532 Tuttlingen (DE)

(57) **Zusammenfassung**

Die vorliegende Erfindung betrifft ein Artikulationsglied für eine oder in einer Auslenkmechanik an einem distalen Ende eines chirurgischen Instruments, insbesondere eines Endoskops, mit einem Grundkörper, der entlang einer Längsachse eine axiale Durchgangsöffnung aufweist, sodass ein Teilabschnitt eines Arbeitskanals des chirurgischen Instruments mit der Durchgangsöffnung ausbildbar oder durch die Durchgangsöffnung hindurchführbar ist, und einem Verbindungsabschnitt, der an zumindest einem axialen Endabschnitt des Grundkörpers angeordnet ist, wobei der Verbindungsabschnitt zum radialen Eingriff mit einem Verbindungsabschnitt eines weiteren Artikulationsglieds ausgebildet ist, sodass eine formschlüssige und gelenkige Verbindung zwischen zwei Artikulationsgliedern ausbildbar ist. Die vorliegende Erfindung betrifft ferner eine Auslenkmechanik sowie ein Verfahren zur Herstellung einer Auslenkmechanik.

## Beschreibung

### GEBIET DER ERFINDUNG

Die vorliegende Erfindung betrifft ein Artikulationsglied für eine oder in einer Auslenkmechanik an einem distalen Ende eines chirurgischen Instruments. Des Weiteren betrifft die Erfindung eine Auslenkmechanik und ein Verfahren zur Herstellung einer Auslenkmechanik.

### TECHNISCHER HINTERGRUND

Eine Auslenkmechanik für endoskopische Instrumente dient zur Abwinkelung eines daran angebrachten Werkzeugs oder Zubehörs, beispielsweise einer Zange, Schere, Klemme, Laserfaser oder Ähnlichem. Die Auslenkmechanik dient daher zur Werkzeugausrichtung an einem distalen Ende des chirurgischen Instruments, insbesondere endoskopischen Instruments, beispielsweise eines Endoskops. Des Weiteren kann ein Zubehör einer Bildgebung, einer Beleuchtung und/oder eine Lenkung, insbesondere durch Lenkmittel wie Zugdrähte oder ähnliches, mithilfe der Auslenkmechanik geführt werden.

Die Auslenkmechanik kann ebenso als Abwinkelungseinheit bzw. Artikulationseinheit bezeichnet werden, die eine Durchgangsöffnung mit einem Arbeitskanal aufweist, durch welche das Zubehör geführt wird. Das Zubehör, wie eine Zange, Schere, Klemme, Laserfaser oder Ähnliches, wird in der Regel durch den Arbeitskanal des endoskopischen Instruments geschoben. Neben diesem Arbeitskanal können eine Licht- und/oder eine bildgebende Einheit fest in dem endoskopischen Instrument verbaut sein. Um ein Abwinkeln zu ermöglichen, ist die Auslenkmechanik aus einzelnen Artikulationsgliedern gefertigt, die gelenkig miteinander verbunden sind. Die Auslenkung bzw. Ausrichtung der Auslenkmechanik kann mit Zugdrähten erfolgen, die ebenso zumindest teilweise durch die Durchgangsöffnung, insbesondere in separaten Öffnungen, geführt werden.

Aufgrund der Vielzahl an möglichem Zubehör sowie strom- und lichtleitender Bauteile sollte die Durchgangsöffnung möglichst geräumig und ohne Hindernisse ausgeführt sein. Durch die Verbindung der einzelnen Artikulationsglieder kann es jedoch zu Querschnittsverengungen im Innern kommen, die den Querschnitt der Durchgangsöffnung abschnittsweise verkleinern. Dies ist beispielsweise der Fall, wenn die einzelnen Artikulationsglieder über Verbindungsmittel, wie beispielsweise Nieten, Bolzen oder Stifte, miteinander verbunden werden, wobei die Verbindungsmittel in den Querschnitt der Durchgangsöffnung hineinragen, und insbesondere kantige Hindernisse darstellen. Weiterhin müssen die Verbindungsmittel mit einem der Artikulationsglieder fest verbunden werden, damit es verliersicher gelagert ist. Dies erfordert zusätzlichen Aufwand und damit verbundene Zeit und Kosten für die Fertigung.

Um dies zu umgehen ist aus der EP 1 604 607 A1 eine Auslenkmechanik bekannt, wobei zwischen einzelnen Artikulationsgliedern scharnierförmige Verbindungen ausgebildet sind, wobei die einzelnen Artikulationsglieder über eine Art bewegliche Schwalbenschwanzverbindung verbunden werden. Dabei ist ein Zapfen in Längsrichtung des Artikulationslieds ausgerichtet und greift in eine Ausnehmung eines benachbarten Artikulationsglieds ein. Die Ausnehmung weist eine halbkreisförmige Form auf, sodass eine Rotation der Artikulationsglieder relativ zueinander ermöglicht wird. Eine derartige Verbindung erfordert jedoch hohen Anforderungen an die Maßgenauigkeit und Präzision, da das Scharniergelenk nur in der Dicke der Wandstärke des Artikulationsglieds ausgebildet ist, und die Artikulationsmechanik bereits an sich einen sehr geringen Querschnitt aufweist. Weiterhin ist die maximale Rotationsbewegung der beiden Glieder durch die Größe der halbkreisförmigen Ausnehmung begrenzt. Besonders große Winkel können dabei kaum umgesetzt werden, da die verbleibende Materialstärke zwischen den Enden der halbkreisförmigen Ausnehmung bei zunehmendem Umfang sehr gering wird.

### ZUSAMMENFASSUNG DER ERFINDUNG

Vor diesem Hintergrund liegt der vorliegenden Erfindung die Aufgabe zugrunde, ein verbessertes Artikulationsglied sowie eine verbesserte Auslenkmechanik sowie deren Herstellung anzugeben.

Erfindungsgemäß wird diese Aufgabe durch ein Artikulationsglied mit den Merkmalen des Patentanspruchs 1, eine Auslenkmechanik mit den Merkmalen des Patentanspruchs 11 und/oder durch ein Verfahren mit den Merkmalen des Patentanspruchs 16 gelöst.

Demgemäß ist vorgesehen:
- Ein Artikulationsglied für eine oder in einer Auslenkmechanik an einem distalen Ende eines chirurgischen Instruments, insbesondere eines Endoskops, mit einem Grundkörper, der entlang einer Längsachse eine axiale Durchgangsöffnung aufweist, sodass ein Teilabschnitt eines Arbeitskanals des chirurgischen Instruments mit der Durchgangsöffnung ausbildbar oder durch die Durchgangsöffnung hindurchführbar ist, und einem Verbindungsabschnitt, der an zumindest einem axialen Endabschnitt des Grundkörpers angeordnet ist, wobei der Verbindungsabschnitt zum radialen Eingriff mit einem Verbindungsabschnitt eines weiteren Artikulationsglieds ausgebildet ist, sodass eine formschlüssige und gelenkige Verbindung zwischen zwei Artikulationsgliedern ausbildbar ist.
- Eine Auslenkmechanik zur Ausrichtung, insbesondere zur Werkzeugausrichtung und/oder zur Ausrichtung von Licht-, Spül- und/oder einer bildgebenden Einheit, an einem distalen Ende eines chirurgischen Instruments, insbesondere eines Endoskops, mit einer Mehrzahl an Artikulationsgliedern, wobei die Artikulationsglieder derart miteinander formschlüssig und gelenkig verbunden sind, dass ein gelenkiger Arbeitskanal des chirurgischen Instruments durch die Durchgangsöffnungen ausgebildet wird oder durch die Durchgangsöffnung ein flexibler Arbeitskanal hindurchführbar ist.
- Ein Verfahren zur Herstellung einer Auslenkmechanik zur Ausrichtung, insbesondere zur Werkzeugausrichtung und/oder zur Ausrichtung von Licht-, Spül- und/oder einer bildgebenden Einheit, an einem distalen Ende eines chirurgischen Instruments, insbesondere eines Endoskops, mit den Schritten: Bereitstellen einer Mehrzahl an Artikulationsgliedern, zumindest teilweises Ineinanderschieben von jeweils benachbarten Artikulationsgliedern, wobei Verbindungabschnitte benachbarter Artikulationsglieder solange relativ zueinander verschoben werden, bis sich die Verbindungsabschnitte in einer Radialrichtung des Grundkörpers derart kontaktieren, dass eine formschlüssige und gelenkige Verbindung der Artikulationsglieder hergestellt wird oder herstellbar ist.

Die der vorliegenden Erfindung zugrundeliegende Erkenntnis besteht darin, dass eine Verbindung der Artikulationsglieder untereinander ohne lose Verbindungsmittel Vorteile bringt und insbesondere eine Einmalverwendung ermöglicht.

Die der vorliegenden Erfindung zugrundeliegende Idee besteht darin, eine Gelenkverbindung einzelne Artikulationsglieder zu schaffen, die allein aus der Geometrie der Artikulationsglieder an sich, d. h. insbesondere einer Geometrie von axialen Endabschnitten bzw. Randbereichen der Artikulationsglieder, möglich ist.

Der Grundkörper ist insbesondere als rohrförmiges oder hülsenförmiges Element ausgebildet, das insbesondere an beiden Endabschnitten offen ausgebildet ist. Dadurch kann eine axiale Durchgangsöffnung entlang der Längsachse des Artikulationsgliedes ausgebildet werden. Die Endabschnitte weisen bevorzugt einen kreisförmigen bzw. ellipsenförmigen Querschnitt auf. Andere Querschnitte sind ebenso denkbar.

An zumindest einem Endabschnitt ist ein Verbindungsabschnitt angeordnet, der zum radialen Eingriff mit einem Verbindungsabschnitt eines weiteren Artikulationsglieds ausgebildet ist. So können beispielsweise eine Vielzahl von gleichartigen Artikulationsgliedern miteinander verbunden werden, um eine Artikulationsmechanik bzw. Auslenkmechanik auszubilden.

Ein radialer Eingriff ist insbesondere eine Kontaktierung zwischen zwei Verbindungsabschnitten benachbarter Artikulationsglieder. Radial kann dabei jede beliebige Richtung in einer Querschnittsebene des Artikulationsglieds bzw. des axialen Endabschnitts bedeuten. So kann an einer beliebigen Position entlang eines Umfangs des axialen Endabschnitts ein Verbindungsabschnitt angeordnet werden. Insbesondere können auch mehrere Verbindungsabschnitte an unterschiedlichen Umfangspositionen ausgebildet werden, die insbesondere jeweils zum radialen Eingriff mit einem Verbindungsabschnitt des benachbarten Artikulationsglieds ausgebildet sind, und insbesondere unabhängig voneinander mit dem benachbarten Artikulationsglied in Eingriff stehen.

Ein radialer Eingriff bietet weiterhin den Vorteil, dass eine relative große Kontaktfläche ausgebildet werden kann, die unabhängig von einem Querschnitt des Grundkörpers bzw. des Artikulationsglieds ist. Dadurch kann eine stabile und robuste Verbindung hergestellt werden, die insbesondere weniger Anforderungen an die Maßhaltigkeit aufweist als eine Verbindung, bei welcher der Kontakt bzw. das Scharniergelenk über eine Kontaktfläche in der Dicke der Wandstärke des Artikulationsglieds ausgebildet ist.

Die Verbindungsabschnitte benachbarter Artikulationsglieder sind insbesondere komplementär zueinander ausgeformt, sodass sich die beiden Verbindungsabschnitte flächig kontaktieren können.

Eine formschlüssige und gelenkige Verbindung zwischen zwei Artikulationsgliedern ermöglicht, dass die Artikulationsglieder relativ zueinander verschwenkt bzw. rotatorisch bewegt werden können. Dabei wird eine Rotationsachse insbesondere durch den Verbindungsabschnitt ausgebildet, beispielsweise quer zu Längsachse.

Das Artikulationsglied ist für eine oder in einer Auslenkmechanik eines chirurgischen Instruments ausgebildet, wobei ein beliebiges Zubehör, wie insbesondere ein Arbeitskanal für ein Werkzeug wie eine Zange, Schere, Laserfaser, Fangkörbchen oder Ähnliches, mithilfe der Auslenkmechanik ausgelenkt bzw. geführt werden kann. Des Weiteren kann ein Zubehör einer Bildgebung, einer Beleuchtung und/oder eine Lenkung, insbesondere durch Lenkmittel wie Zugdrähte sowie ein Spül-, Saug- und/oder Arbeitskanal oder ähnliches, mithilfe der Auslenkmechanik geführt werden.

Die Vielzahl an Zubehör muss dabei durch die Durchgangsöffnung des Artikulationsglieds bzw. der Auslenkmechanik geführt werden. Dabei ist es vorteilhaft, wenn der Innendurchmesser der Durchgangsöffnung zum einen kontinuierlich konstant, und zum anderen möglichst ohne "Hindernisse" ausgebildet ist. Insbesondere bei sehr kleinen Durchmessern der Artikulationsglieder, wie beispielsweise von Durchmessern kleiner oder gleich 3 mm, sind daher hohe Anforderungen an die Gelenkverbindung zwischen benachbarten Artikulationsgliedern gesetzt, sodass diese platzsparend ausgebildet werden sollten. Die vorgeschlagene formschlüssige und gelenkige Verbindung bietet den Vorteil, dass diese kaum, insbesondere bis gar nicht, in den Innenquerschnitt des Grundelements eingreift. Insbesondere ist eine Überstand des Verbindungsabschnitts in das Innere des Grundelements derart gering, dass der Arbeitskanal dadurch nicht gestört wird. Das Artikulationsglied bietet daher insbesondere für besonders kleine Durchmesser des Grundkörpers Vorteile, insbesondere für Durchmesser im Millimeterbereich, bevorzugt von 3 mm oder kleiner.

Bei dem chirurgischen Instrument kann es sich insbesondere um ein Endoskop handeln. Ebenso ist das Artikulationsglied für andere chirurgische Instrumente bzw. endoskopische Instrumente geeignet, bei welchen eine Miniaturisierung der Auslenkmechanik von Vorteil ist.

Vorteilhafterweise kann daher eine nietfreie Verbindung zwischen Artikulationsgliedern erreicht werden, die insbesondere allein aus einem Formschluss zwischen axialen Endabschnitten erreicht werden kann. Die Artikulationsglieder können einfach zusammengesteckt werden, wobei durch elastisches Umformen zumindest eines Endabschnitts eine Gelenkverbindung ausgebildet werden kann. Die Endabschnitte benachbarter Artikulation können daher miteinander verrasten bzw. einschnappen, wodurch eine Gelenkverbindung ausgebildet werden kann.

Vorteilhafte Ausgestaltungen und Weiterbildungen ergeben sich aus den weiteren Unteransprüchen sowie aus der Beschreibung unter Bezugnahme auf die Figuren der Zeichnung.

In einer bevorzugten Ausführungsform kann der Verbindungsabschnitt elastisch ausgebildet sein, wobei durch eine elastische Auslenkung in radialer Richtung die formschlüssige Verbindung herstellbar ist. Elastisch bedeutet insbesondere, dass der Verbindungsabschnitt in zwei entgegengesetzte Richtungen elastisch ausgelenkt werden kann, sodass sich der Verbindungsabschnitt elastisch in die Ausgangsposition zurück verformt, wenn dieser mit dem Verbindungsabschnitt eines benachbarten Artikulationsglieds verbunden ist. Das Artikulationsglied ist daher bevorzugt aus einem Metall ausgeformt, und kann insbesondere aus einem Rohr herausgeschnitten hergestellt sein.

In einer bevorzugten Ausführungsform kann der Verbindungsabschnitt zumindest eine Lasche aufweisen. Als Lasche ist insbesondere ein Abschnitt zu verstehen, der an dem axialen Endabschnitt herausragt. Die Lasche kann dabei beispielsweise eine Gelenkachse definieren, sodass benachbarte Artikulationsglieder geführt gegeneinander ausgelenkt werden können.

In einer bevorzugten Ausführungsform kann zumindest eine Lasche in einer Längsrichtung des Artikulationsglieds den Grundkörper überragen und elastisch in einer Radialrichtung auslenkbar sein. Vorteilhafterweise wird dabei der Innendurchmesser des Grundkörpers durch die Lasche nicht, insbesondere nur wenig beeinflusst. Die Lasche ist insbesondere derart ausgerichtet, dass diese eine in einer Tangentialrichtung bzw. in einer Umfangsrichtung ausgerichtete flächige Oberfläche aufweist. Dadurch wird eine zweidimensionale Fläche ausgeformt, die zum radialen Eingriff genutzt werden kann. Eine Gelenkachse kann innerhalb und quer zu dieser zweidimensionalen Fläche ausgeformt sein.

Insbesondere kann die Lasche in einen radialen Eingriff mit einer weiteren Lasche eines benachbarten Artikulationsglieds gebracht werden. Bei einem radialen Eingriff können sich die beiden Laschen über jeweilige tangentiale und/oder in Umfangsrichtung verlaufende Oberflächen der jeweiligen Lasche kontaktieren.

In einer bevorzugten Ausführungsform kann die zumindest eine Lasche kuppelförmig ausgebildet sein, wobei die formschlüssige und gelenkige Verbindung durch Kontakt der Lasche mit einer zu der kuppelförmigen Lasche komplementären Lasche eines weiteren Artikulationsglieds ausbildbar ist. Insbesondere kann die Kuppelform allein durch einen Umformprozess hergestellt werden, ohne das Material entnommen oder Material hinzugefügt werden muss. Die Kuppelform bietet weiterhin einen flächigen Kontakt zu einem benachbarten Artikulationsglied, der sich nach einschieben bzw. einschnappen benachbarter Artikulationsglieder selbst ausrichtet. Kuppelförmig bedeutet daher insbesondere, dass die Lasche eine Verwölbung aufweist.

In einer bevorzugten Ausführungsform kann die kuppelförmige Lasche eine in einer Radialrichtung des Grundkörpers konvexe oder konkave Kuppel aufweisen. Dadurch kann eine definierte Gelenkachse durch einen Mittelpunkt des Grundkörpers umgesetzt werden. Bei einer konvex geformten Kuppel ist die Kuppel insbesondere in Richtung einer Außenoberfläche des Grundkörpers ausgeformt, wodurch das Innere des Grundkörpers nicht beeinflusst wird. Bei einer konkav geformten Kuppel ist die Kuppel insbesondere in Richtung einer Innenoberfläche zu einem Mittelpunkt des Grundkörpers ausgerichtet. Dadurch ragt die Kuppel zwar minimal in die Durchgangsöffnung hinein, stellt durch die stetig geformte Oberfläche der gewölbten Kuppel jedoch kein kantiges Hindernis dar, an welcher sich ein Zubehör verhaken kann. Des Weiteren ist es ausreichend, wenn die Kuppel nur eine geringe Höhe bis zum Scheitelpunkt aufweist, um bereits eine Gelenkverbindung auszuformen.

In einer bevorzugten Ausführungsform können an zumindest einem Endabschnitt zwei Laschen vorgesehen sein, die insbesondere gegenüberliegend an einem Querschnitt des Endabschnitts und/oder gespiegelt zueinander ausgebildet sind. Jede der Laschen kann wie oben dargestellt ausgeformt sein. So kann beispielsweise eine definierte Gelenkachse zwischen den beiden Laschen umgesetzt werden.

In einer bevorzugten Ausführungsform kann an zwei Endabschnitten, die am Grundkörper gegenüberliegend angeordnet sind, jeweils ein Verbindungsabschnitt angeordnet sein, sodass das Artikulationsglied an jedem Endabschnitt mit einem weiteren Artikulationsglied verbindbar ist. Dadurch kann eine Vielzahl von Artikulationsgliedern miteinander verbunden werden, wobei die Artikulationsglieder alle baugleich ausgeformt werden können. Beispielsweise können an den Endabschnitten der Auslenkmechanik sogenannte Endglieder eingesetzt werden, die lediglich an einem Endabschnitt einen Verbindungsabschnitt aufweisen, und an dem anderen Endabschnitt ohne Verbindungsabschnitt ausgeformt sind.

In einer bevorzugten Ausführungsform kann die Lasche zum gelenkigen Eingriff mit einem Verbindungsabschnitt des weiteren Artikulationsglieds auf einer vorbestimmten Gelenkachse ausgebildet sein. Die Gelenkachse kann insbesondere durch den Mittelpunkt des Grundkörpers verlaufen.

In einer bevorzugten Ausführungsform kann die Durchgangsöffnung zur Durchführung und definierten Aufnahme von Lenkmitteln, insbesondere Lenkseilen oder Lenkdrähten, ausgebildet sein. Dazu können aus dem Grundkörper Formteile heraus geformt werden, die als Führungselemente für die Lenkmittel dienen können. Die Formteile können insbesondere zumindest teilweise mit dem Grundkörper verbunden sein und einen definierten Führungskanal innerhalb des Innenquerschnitt des Grundkörpers ausformen.

In einer bevorzugten Ausführungsform der Auslenkmechanik können die Verbindungsabschnitte benachbarter Artikulationsglieder in einer Ebene versetzt zueinander angeordnet sein, wobei diese in einem verbundenen Zustand übereinander angeordnet sind und radial ineinander eingreifen. In einer Ebene versetzt bedeutet insbesondere, dass die Verbindungsabschnitte an unterschiedlichen radialen Abständen angeordnet sind. So kann ein Verbindungsabschnitt näher an einem Mittelpunkt des Grundkörpers liegen als ein Verbindungsabschnitt eines benachbarten Artikulationsglieds. Dadurch können die Verbindungsabschnitte übereinander geschoben werden, wenn die Artikulationsglieder miteinander verbunden werden. Insbesondere können die Verbindungsabschnitte dabei miteinander verrasten oder einschnappen, sodass eine formschlüssige und gelenkige Verbindung erfolgt.

Ein Artikulationsglied kann dabei an zwei gegenüberliegenden Endabschnitten Verbindungsabschnitte aufweisen, die in einer Ebene versetzt zueinander angeordnet sind. Dadurch können baugleiche Artikulationsglieder zur Ausbildung einer zusammenhängenden Auslenkmechanik miteinander verbunden werden.

Unabhängig von der Ausführungsform sind die Laschen aneinander angrenzender Artikulationsglieder insbesondere parallel zueinander ausgerichtet und/oder komplementär zueinander ausgeformt.

In einer bevorzugten Ausführungsform der Auslenkmechanik können die Verbindungsabschnitte jeweils zumindest eine Lasche aufweisen, wobei zumindest eine der Laschen elastisch in eine Radialrichtung des Grundkörpers elastisch auslenkbar ist und in einem verbundenen Zustand die Laschen miteinander verrastet sind. Insbesondere ist die Lasche in zwei entgegengesetzte Richtungen in Radialrichtung elastisch verformbar, sodass diese selbstrückstellend ausgebildet ist.

In einer bevorzugten Ausführungsform der Auslenkmechanik können die Laschen benachbarter Artikulationsglieder eine komplementäre Form aufweisen, insbesondere eine komplementäre Kuppelform. Dadurch kann eine Gelenkachse ausgeformt werden, um welche die beiden Artikulationsglieder beliebig relativ zueinander rotatorischen bewegt werden können. Der Rotationswinkel wird dabei nicht durch die Lasche bzw. die Kuppelform begrenzt, sondern allein durch die Form des Grundkörpers, wobei durch Seitenabschnitte an den axialen Endabschnitten des Grundkörpers festgelegt wird, wie weit die Artikulationsglieder relativ zueinander bewegt werden können, bis diese sich berühren.

In einer bevorzugten Ausführungsform der Auslenkmechanik kann die Auslenkmechanik zur Einmalverwendung ausgebildet sein. Dies wird insbesondere dadurch erreicht, dass die einzelnen Artikulationsglieder der Auslenkmechanik zum einen kostengünstig und ohne großen Zeitaufwand hergestellt werden können. Zum anderen ist die Verbindung der Artikulationsglieder untereinander durch Wegfallen von weiteren Verbindungsmitteln mit geringem Zeitaufwand umsetzbar, und kann durch einfaches Einschnappen bzw. Verrasten der Verbindungsabschnitte umgesetzt werden. Dabei kann die Auslenkmechanik vor Ort werkzeugfrei montiert werden.

Die Einmalverwendung, auch Single Use genannt, bietet dabei mehrere Vorteile. Zum einen wird von vornherein eine Sterilität gewährleistet und insbesondere Kreuzkontaminationen verhindert. Weiterhin besteht kein Aufwand für die Reinigung sowie kein Reparaturaufwand beschädigter Elemente. Insbesondere bei Endoskopen, die sehr fragil sind, kann es schnell zu Beschädigungen kommen. Eine Auslenkmechanik zur Einmalverwendung bietet daher den Vorteil, dass aufgrund der kostengünstigen Herstellung auf eine Reparatur verzichtet werden kann. Im Gegensatz zu wiederverwendbaren Endoskopen werden Einwegendoskope daher nach dem Gebrauch entsorgt. Auf eine Reparatur beschädigter Produkte kann demnach verzichtet werden. Weiterhin werden meist mehrere chirurgische Instrumente parallel verwendet. Dadurch muss eine hohe Verfügbarkeit bereitgestellt werden, um gleichzeitig mehrere Auslenkmechaniken nutzen zu können. Ist jede Auslenkmechanik für sich kostengünstig, so können mehrere Auslenkmechaniken vorgehalten werden, ohne eine finanzielle Belastung darzustellen.

In einer bevorzugten Ausführungsform des Verfahrens kann die eine Verbindung von zwei benachbarten Artikulationsgliedern durch einen auf das Ineinanderschieben folgenden Umformungsschritt ausgeformt werden, wobei sich kontaktierende, parallel übereinander geschobene Verbindungsbereiche komplementär umgeformt werden. So können beispielsweise nach dem Zusammenschieben zwei Verbindungsabschnitte, insbesondere zwei Laschen, die parallel zueinander angeordnet sind, kuppelförmige umgeformt werden, um eine Gelenkachse zu definieren. Dadurch kann beispielsweise ohne großen Aufwand die Genauigkeit und Konzentrizität der Verbindung beziehungsweise der Gelenkachse umgesetzt werden.

In einer bevorzugten Ausführungsform des Verfahrens kann zumindest ein Verbindungsabschnitt jeweils eines Artikulationsglieds elastisch ausgebildet sein, wobei durch eine elastische Auslenkung beim Ineinanderschieben die Verbindungsabschnitte miteinander verrasten. In einer derartigen Ausführung können die Verbindungsabschnitte bzw. Laschen bereits vor dem Ineinanderschieben kuppelförmig ausgeformt sein, sodass sich die Verbindungsabschnitte bzw. Laschen beim Ineinanderschieben mithilfe der konvexen oder konkaven Kuppelform zentrieren, selbst ausrichten und formschlüssig sowie gelenkig miteinander verbinden.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: eine isometrische Darstellung einer Auslenkmechanik;
- Fig. 2: eine Detailansicht aus Fig. 1;
- Fig. 3: eine weitere Detailansicht der Ausführung nach Fig. 2;
- Fig. 4: eine weitere Detailansicht der Ausführung nach Fig. 2;
- Fig. 5: ein Artikulationsglied in einer isometrischen Darstellung;
- Fig. 6: eine Schnittdarstellung durch ein Artikulationsglied;
- Fig. 7: eine Schnittdarstellung durch die Ausführung nach Fig. 1;
- Fig. 8: eine Schnittdarstellung einer weiteren Ausführungsform einer Auslenkmechanik;
- Fig. 9: eine Seitenansicht der Ausführungsform nach Fig. 8;
- Fig. 10: eine Detailansicht aus Fig. 9;
- Fig. 11: eine Draufsicht der Ausführungsform nach Fig. 9;
- Fig. 12: eine weitere Ansicht der Ausführungsform nach Fig. 9; und
- Fig. 13: eine Schnittdarstellung durch ein Artikulationsglied für eine Auslenkmechanik nach Fig. 9.

Die beiliegenden Figuren der Zeichnung sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausgeführt ist - jeweils mit denselben Bezugszeichen versehen.

### BESCHREIBUNG VON AUSFÜHRUNGSBEISPIELEN

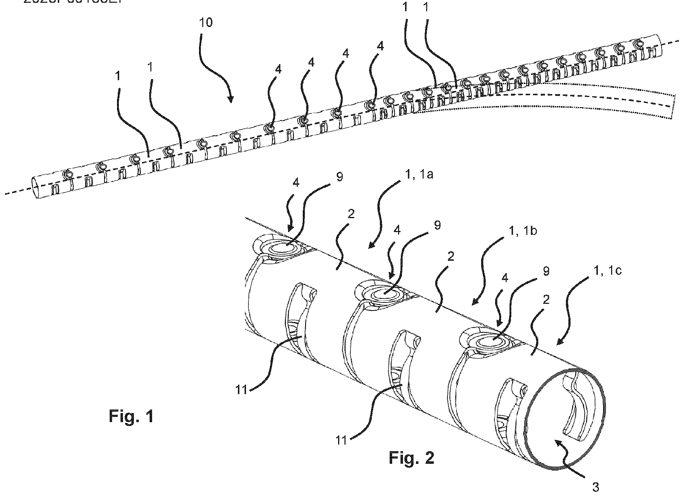

Fig. 1 zeigt eine isometrische Darstellung einer Auslenkmechanik 10. Die Auslenkmechanik 10 ist aus mehreren Artikulationsgliedern 1 ausgebildet, die über Verbindungsabschnitte 4 miteinander verbunden sind. Dadurch kann die Auslenkmechanik 10 gelenkig ausgeformt sein, sodass diese von einer Längsachse ausgelenkt werden kann, wie durch die nach unten geneigte und gestrichelte Darstellung beispielhaft gezeigt. Die Auslenkmechanik 10 kann unterschiedlich lange Artikulationsglieder 1 aufweisen, wodurch der Radius einer Auslenkung bzw. eine rotatorischen Bewegung beeinflusst werden kann.

Fig. 2 zeigt eine Detailansicht aus Fig. 1. Dabei sind mehrere Artikulationsglieder 1a, 1b, 1c gezeigt, die jeweils einen Grundkörper 2 aufweisen. Der Grundkörper 2 ist insbesondere rohrförmig bzw. hülsenförmig ausgeformt, sodass eine Durchgangsöffnung 3 ausgebildet werden kann. An dem Grundkörper 2 können weiterhin Formteile 11 teilweise herausgeschnitten und derart verformt sein, dass weitere Führungskanäle 12 innerhalb der Durchgangsöffnung 3 ausgebildet werden. Die Führungskanäle 12 können insbesondere zur Durchführung von einer Lenkmechanik genutzt werden.

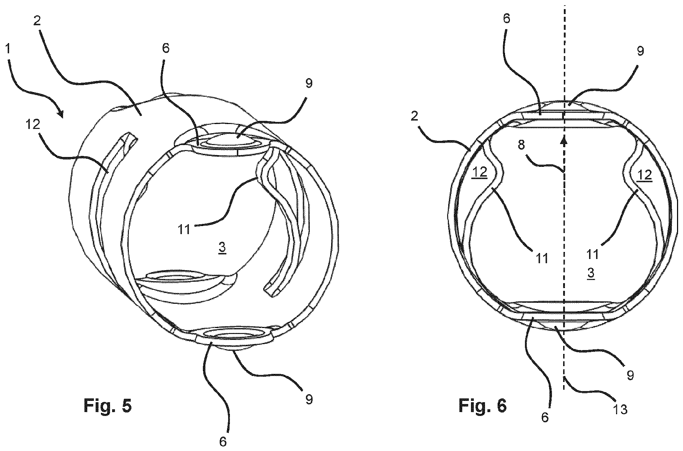

Die dargestellten Artikulationsglieder sind in einem verbundenen Zustand gezeigt, wobei die Verbindungsabschnitte 4 benachbarter Artikulationsglieder miteinander verbunden sind. In der dargestellten Ausführungsform weisen die Verbindungsabschnitte 4 jeweils eine Kuppel 9 auf, die eine Gelenkachse 13 definiert, diese ist in Fig. 6 gezeigt. Die Verbindungsabschnitte 4 aneinander angrenzender Artikulationsglieder 1 sind insbesondere parallel zueinander ausgerichtet und/oder komplementär zueinander ausgeformt.

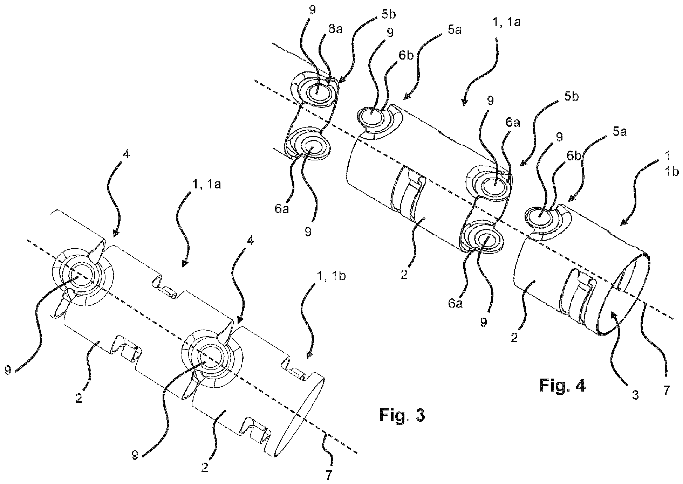

Fig. 3 zeigt eine weitere Detailansicht der Ausführung nach Fig. 2. Die Artikulationsglieder 1a, 1b berühren sich an den Verbindungsabschnitten 4 lediglich im Bereich der Kuppel 9, wobei der restliche axiale Endabschnitt des Artikulationsglieds 1a beanstandet zu dem axialen Endabschnitt des Artikulationsglieder 1b angeordnet ist. Dadurch kann eine Auslenkung von der Längsachse 7 erreicht werden, da die Artikulationsglieder 1a, 1b eine rotatorische Bewegung relativ zueinander ausüben können. Die Kuppel 9 dient insbesondere zur Führung der rotatorischen Bewegung.

Fig. 4 zeigt eine weitere Detailansicht der Ausführung nach Fig. 2. Die Artikulationsglieder 1 sind vor dem Ineinanderschieben dargestellt. Das Artikulationsglied 1a weist an zwei gegenüberliegend angeordneten, axialen Endabschnitten 5a, 5b jeweils einen Verbindungsabschnitt auf, der in dieser Ausführungsform als Lasche 6a bzw. Lasche 6b ausgebildet ist. In jeder Lasche 6a, 6b ist eine Kuppel 9 ausgeformt, die in dieser Ausführungsform konvex in Radialrichtung ausgerichtet ist. Dies ist beispielsweise aus Figur 6 ersichtlich.

Fig. 5 zeigt ein Artikulationsglied 1 in einer isometrischen Darstellung und Fig. 6 eine Schnittdarstellung durch ein Artikulationsglied 1. Abweichend zu der dargestellten Ausführungsform können die Laschen 6 auch an anderen Umfangsabschnitten des Grundkörpers 2 bzw. des Verbindungabschnitts 4 angeordnet sein. Ebenso ist denkbar, dass lediglich eine Lasche 6 an einem axialen Endabschnitt ausgeformt ist. Da die Kuppel 9 in Richtung einer Außenoberfläche des Grundkörpers 2 gewölbt ist, ragt diese nicht in den inneren Querschnitt der Durchgangsöffnung 3 hinein. Dadurch wird der innere Querschnitt nicht durch den Verbindungsabschnitt 4 beeinflusst. Insbesondere kann dadurch ein nahezu konstanter und gleichförmiger Innenquerschnitt entlang der Längsachse 7 erreicht werden.

In einer weiteren Ausführungsform können Formteile 11 vorgesehen sein, die aus dem Grundkörper 2 herausgeformt werden, jedoch weiterhin mit dem Grundkörper 2 verbunden sind. Dadurch können beispielsweise Führungskanäle 12 innerhalb der Durchgangsöffnung 3 als separate Öffnungen ausgeformt werden, die insbesondere zur Führung einer Lenkmechanik dienen können.

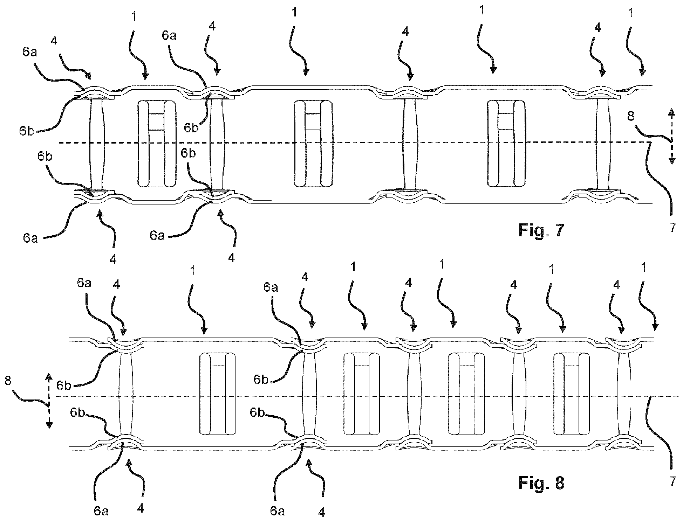

Fig. 7 zeigt eine Schnittdarstellung durch die Ausführung nach Fig. 1. Dabei ist erkennbar, wie die beiden kuppelförmigen Laschen 6a, 6b ineinander eingreifen.

Die Laschen 6a, 6b sind insbesondere komplementär zueinander ausgeformt, sodass sich die beiden Laschen 6a, 6b flächig kontaktieren können.

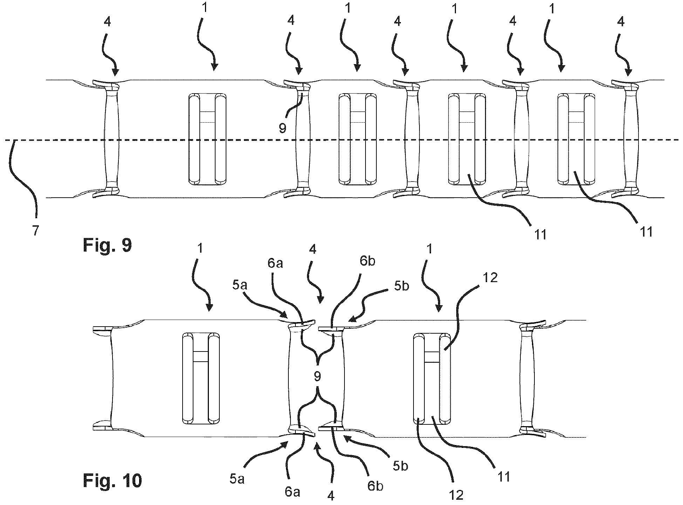

Fig. 8 zeigt eine Schnittdarstellung einer weiteren Ausführungsform einer Auslenkmechanik 10. In dieser Ausführungsform sind die Laschen 6a, 6b ebenso kuppelförmig ausgeformt, wobei die Kuppel konkav bezüglich der Radialrichtung 8 ausgeformt ist. Die Laschen 6a, 6b sind daher in Richtung eines Mittelpunkts der Durchgangsöffnung 3 gewölbt, sodass diese minimal in die Durchgangsöffnung 3 hineinragen. Aufgrund der gewölbten Form mit einer stetigen Oberfläche entsteht dabei jedoch kein Hindernis für ein Zubehör. Vielmehr kann die stetige Kuppelform zur Führung des Zubehörs dienen, wenn dieses entlang der Durchgangsöffnung 3 und in Richtung der Längsachse 7 verschoben wird. Eine Seitenansicht der Ausführungsform nach Fig. 8 ist in Fig. 9 gezeigt.

Fig. 10 zeigt eine Detailansicht der Ausführungsform nach Fig. 9, wobei die beiden Artikulationsglieder 1 vor dem Zusammenbau, d. h. vor dem Ineinanderschieben dargestellt sind. Dabei ist erkennbar, dass die beiden Kuppeln 9 von aneinander angrenzenden Laschen 6a, 6b komplementär ausgeformt sind. Es ist ebenso erkennbar, dass die mit den Bezugszeichen versehenen Laschen 6a des links dargestellten Artikulationsglieds 1 in einer Ebene versetzt zu den Laschen angeordnet sind, die sich an einem gegenüberliegenden axialen Endabschnitt (ganz links in der Darstellung) an demselben Artikulationsglieder 1 befinden. Durch eine derartige Ausführung können besonders vorteilhaft baugleiche Artikulationsglieder 1 ineinandergeschoben werden.

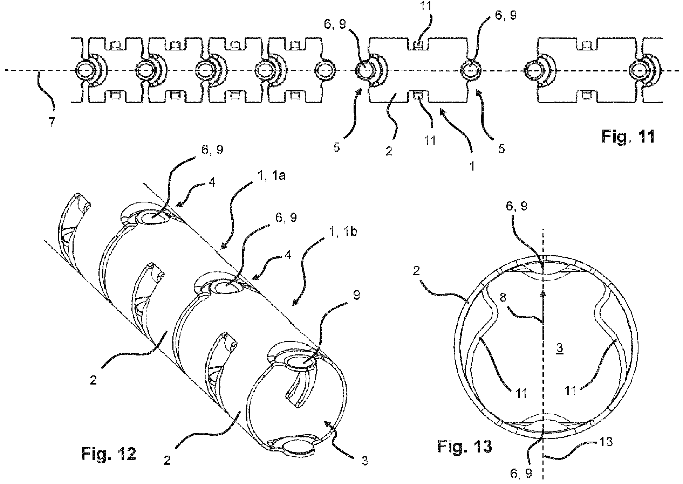

Weitere Darstellungen der Ausführungsform nach Fig. 8 sind in den Figuren 11 bis 13 gezeigt.

Unabhängig von der Ausführungsform können die Artikulationsglieder 1 aus einem kompletten Rohr hergestellt bzw. ausgeschnitten werden. Dies kann insbesondere durch Laserschneiden, Plasmaschneiden, Wasserstrahlschneiden, Stanzen oder Ähnliches erfolgen. Durch einen Umformprozess können die kuppelförmigen Laschen ausgeformt werden. Da das Rohr insbesondere aus einem Metallmaterial besteht, sind die Laschen 6 bzw. die Verbindungsabschnitte 4 elastisch ausgeformt, sodass die Artikulationsglieder 1 ineinander eingeschoben werden können, wobei durch eine elastische Verformung eine Verbindung hergestellt werden kann.

Die Auslenkmechanik 10 kann beispielsweise für unterschiedliche chirurgische Instrumente, so insbesondere für Endoskope und auch für Katheter, eingesetzt werden. Dabei ist die Auslenkmechanik 10 insbesondere für kleine Durchmesser der Artikulationsglieder 1 ausgelegt. So können insbesondere Durchmesser von 2-5 mm, insbesondere von 3 mm, bevorzugt von weniger als 3 mm, umgesetzt werden.

Die Auslenkmechanik 10 kann beispielsweise auch vor Ort direkt montiert werden, da die Montage durch einfaches verrasten bzw. verschnappen der einzelnen Artikulationsglieder 1 erreicht werden kann.

Die kuppelförmigen Laschen können entweder vor dem Ineinanderschieben oder nach dem Ineinanderschieben ausgeformt werden. Vorteilhafterweise werden die kuppelförmigen Laschen vor dem Ineinanderschieben ausgeformt, sodass diese bereits beim Ineinanderschieben verschnappen. Die Verbindungsgeometrien können sich dabei elastisch in radiale Richtung verformen, wodurch eine formschlüssige und gelenkige Verbindung hergestellt wird. Der Formschluss bietet dabei den Vorteil, dass auf weitere Verbindungsmittel, wie insbesondere Nieten, Bolzen oder dergleichen, verzichtet werden kann.

Die Auslenkmechanik 10 bzw. die Artikulationsglieder 1 eignen sich besonders zu Einmalverwendung, d. h. für eine Single Use Anwendung, da diese kostengünstig hergestellt und zeitsparend miteinander verbunden werden können.

Obwohl die vorliegende Erfindung anhand bevorzugter Ausführungsbeispiele vorstehend vollständig beschrieben wurde, ist sie darauf nicht beschränkt, sondern auf vielfältige Art und Weise modifizierbar.

### Bezugszeichenliste

- 1: Artikulationsglied
- 2: Grundkörper
- 3: Durchgangsöffnung
- 4: Verbindungsabschnitt
- 5: Endabschnitt
- 6: Lasche
- 7: Längsrichtung
- 8: Radialrichtung
- 9: Kuppel
- 10: Auslenkmechanik
- 11: Formteil
- 12: Öffnung
- 13: Gelenkachse

## Patentansprüche

1. Artikulationsglied (1) für eine oder in einer Auslenkmechanik (10) an einem distalen Ende eines chirurgischen Instruments, insbesondere eines Endoskops, mit:
einem Grundkörper (2), der entlang einer Längsachse (7) eine axiale Durchgangsöffnung (3) aufweist, sodass ein Teilabschnitt eines Arbeitskanals des chirurgischen Instruments mit der Durchgangsöffnung (3) ausbildbar oder durch die Durchgangsöffnung hindurchführbar ist; und
einem Verbindungsabschnitt (4), der an zumindest einem axialen Endabschnitt (5) des Grundkörpers (2) angeordnet ist, wobei der Verbindungsabschnitt (4) zum radialen Eingriff mit einem Verbindungsabschnitt (4) eines weiteren Artikulationsglieds (1) ausgebildet ist, sodass eine formschlüssige und gelenkige Verbindung zwischen zwei Artikulationsgliedern (1) ausbildbar ist.

2. Artikulationsglied (1) nach Anspruch 1,
**dadurch gekennzeichnet,**
**dass** der Verbindungsabschnitt (4) elastisch ausgebildet ist, wobei durch eine elastische Auslenkung in radialer Richtung die formschlüssige Verbindung herstellbar ist.

3. Artikulationsglied (1) nach einem der vorstehenden Ansprüche,
**dadurch gekennzeichnet,**
**dass** der Verbindungsabschnitt (4) zumindest eine Lasche (6) aufweist.

4. Artikulationsglied (1) nach Anspruch 3,
**dadurch gekennzeichnet,**
**dass** die zumindest eine Lasche (6) in einer Längsrichtung (7) des Artikulationsglieds (1) den Grundkörper (2) überragt und elastisch in einer Radialrichtung (8) auslenkbar ist.

5. Artikulationsglied (1) nach einem der Ansprüche 3 oder 4,
**dadurch gekennzeichnet,**
**dass** die zumindest eine Lasche (6) kuppelförmig ausgebildet ist, wobei die formschlüssige und gelenkige Verbindung durch Kontakt der Lasche (6) mit einer zu der kuppelförmigen Lasche komplementären Lasche (6) eines weiteren Artikulationsglieds (1) ausbildbar ist.

6. Artikulationsglied (1) nach Anspruch 5,
**dadurch gekennzeichnet,**
**dass** die kuppelförmige Lasche (6) eine in einer Radialrichtung (8) des Grundkörpers (2) konvexe oder konkave Kuppel (9) aufweist.

7. Artikulationsglied (1) nach einem der Ansprüche 3 bis 6,
**dadurch gekennzeichnet,**
**dass** an zumindest einem Endabschnitt (5) zwei Laschen (6) vorgesehen sind, die insbesondere gegenüberliegend an einem Querschnitt des Endabschnitts (5) und/oder gespiegelt zueinander ausgebildet sind.

8. Artikulationsglied (1) nach einem der vorstehenden Ansprüche,
**dadurch gekennzeichnet,**
**dass** an zwei Endabschnitten (5a, 5b), die am Grundkörper (2) gegenüberliegend angeordnet sind, jeweils ein Verbindungsabschnitt (4) angeordnet ist, sodass das Artikulationsglied (1) an jedem Endabschnitt (5a, 5b) mit einem weiteren Artikulationsglied (1) verbindbar ist.

9. Artikulationsglied (1) nach einem der Ansprüche 3 bis 8,
**dadurch gekennzeichnet,**
**dass** die Lasche (6) zum gelenkigen Eingriff mit einem Verbindungsabschnitt (4) des weiteren Artikulationsglieds (1) auf einer vorbestimmten Gelenkachse (13) ausgebildet ist.

10. Artikulationsglied (1) nach einem der vorstehenden Ansprüche,
**dadurch gekennzeichnet,**
**dass** die Durchgangsöffnung (3) zur Durchführung und definierten Aufnahme von Lenkmitteln, insbesondere Lenkseilen oder Lenkdrähten, ausgebildet ist.

11. Auslenkmechanik (10) zur Ausrichtung, insbesondere zur Werkzeugausrichtung und/oder zur Ausrichtung von Licht-, Spül- und/oder einer bildgebenden Einheit, an einem distalen Ende eines chirurgischen Instruments, insbesondere eines Endoskops, mit:
einer Mehrzahl an Artikulationsgliedern (1a, 1b) nach einem der Ansprüche 1 bis 10, wobei die Artikulationsglieder (1a, 1b) derart miteinander formschlüssig und gelenkig verbunden sind, dass ein gelenkiger Arbeitskanal des chirurgischen Instruments durch die Durchgangsöffnungen ausgebildet wird oder durch die Durchgangsöffnung ein flexibler Arbeitskanal hindurchführbar ist.

12. Auslenkmechanik (10) nach Anspruch 11,
**dadurch gekennzeichnet,**
**dass** Verbindungsabschnitte (4) benachbarter Artikulationsglieder (1a, 1b) in einer Ebene versetzt zueinander angeordnet sind, wobei diese in einem verbundenen Zustand übereinander angeordnet sind und radial ineinander eingreifen.

13. Auslenkmechanik (10) nach Anspruch 11 oder 12,
**dadurch gekennzeichnet,**
**dass** die Verbindungsabschnitte (4) jeweils zumindest eine Lasche (6) aufweisen, wobei zumindest eine der Laschen (6) elastisch in eine Radialrichtung (8) des Grundkörpers (2) elastisch auslenkbar ist und in einem verbundenen Zustand die Laschen (6) miteinander verrastet sind.

14. Auslenkmechanik (10) nach einem Anspruch 13,
**dadurch gekennzeichnet,**
**dass** die Laschen (6) benachbarter Artikulationsglieder (1) eine komplementäre Form aufweisen, insbesondere eine komplementäre Kuppelform.

15. Auslenkmechanik (10) nach einem der Ansprüche 10 bis 14,
**dadurch gekennzeichnet,**
**dass** die Auslenkmechanik (10) zur Einmalverwendung ausgebildet ist.

16. Verfahren zur Herstellung einer Auslenkmechanik (10) zur Ausrichtung, insbesondere zur Werkzeugausrichtung und/oder zur Ausrichtung von Licht-, Spül- und/oder einer bildgebenden Einheit, an einem distalen Ende eines chirurgischen Instruments, insbesondere eines Endoskops, mit den Schritten:
Bereitstellen einer Mehrzahl an Artikulationsgliedern (1a, 1b) nach einem der Ansprüche 1 bis 10,
zumindest teilweises Ineinanderschieben von jeweils benachbarten Artikulationsgliedern (1a, 1b), wobei Verbindungabschnitte (4) benachbarter Artikulationsglieder (1a, 1b) solange relativ zueinander verschoben werden, bis sich die Verbindungsabschnitte (4) in einer Radialrichtung (8) des Grundkörpers (2) derart kontaktieren, dass eine formschlüssige und gelenkige Verbindung der Artikulationsglieder (1a, 1b) hergestellt wird oder herstellbar ist.

17. Verfahren nach Anspruch 16,
**dadurch gekennzeichnet,**
**dass** eine Verbindung von zwei benachbarten Artikulationsgliedern (1a, 1b) durch einen auf das Ineinanderschieben folgenden Umformungsschritt ausgeformt wird, wobei sich kontaktierende, parallel übereinander geschobene Verbindungsbereiche (4) komplementär umgeformt werden.

18. Verfahren nach Anspruch 16,
**dadurch gekennzeichnet,**
**dass** zumindest ein Verbindungsabschnitt (4) jeweils eines Artikulationsglieds (1) elastisch ausgebildet ist, wobei durch eine elastische Auslenkung beim Ineinanderschieben die Verbindungsabschnitte (4) miteinander verrasten.
